# EUROPEAN PATENT SPECIFICATION

(11) **EP 4 268 637 B1**
(45) Date of publication and mention of the grant of the patent: **12.08.2026**
(21) Application number: 21909057.8
(22) Date of filing: 29.11.2021
(51) Int. Cl.: A24F 40/46, A24F 40/40, A24F 40/05, A61M 11/00, A61M 11/04, A61M 15/00, B05B 17/00, H05B 3/04, H05B 3/14, H05B 3/28, A24F 40/10

(54) **ATOMIZER AND ELECTRONIC ATOMIZATION DEVICE**
ZERSTÄUBER UND ELEKTRONISCHE ZERSTÄUBUNGSVORRICHTUNG
ATOMISEUR ET DISPOSITIF D'ATOMISATION ÉLECTRONIQUE

(30) Priority: 22.12.2020 CN 202011525441
(43) Date of publication of application: 01.11.2023
(73) Proprietor: Shenzhen Smoore Technology Limited, Shenzhen, Guangdong 518102 (CN)
(72) Inventor: ZHOU, Hongming, Shenzhen, Guangdong 518102 (CN); LI, Hong, Shenzhen, Guangdong 518102 (CN); LI, Huanxi, Shenzhen, Guangdong 518102 (CN); PENG, Ce, Shenzhen, Guangdong 518102 (CN)
(74) Representative: Ing. C. Corradini & C. S.R.L.
(86) International application number: PCT/CN2021/133814
(87) International publication number: WO 2022/135058

(56) References cited:
- EP-B1- 3 479 705
- WO-A1-2017/076590
- WO-A1-2018/001106
- WO-A1-2018/053955
- WO-A1-2020/209112
- CN-A- 104 983 078
- CN-A- 108 013 512
- CN-A- 109 261 428
- CN-A- 112 617 297
- CN-U- 205 922 888
- US-A1- 2019 216 135

## Description

### TECHNICAL FIELD

This application relates to the field of atomization technologies. In particular, the invention relates to an atomizer and an electronic atomization device including the atomizer.

### BACKGROUND

Atomizer usually includes an ultrasonic atomization piece, and an atomization hole is provided in the ultrasonic atomization piece. When vibrating at a high frequency, the ultrasonic atomization piece may atomize liquid in the atomization hole to form liquid vapor, and the liquid vapor is sprayed from the atomization hole to be absorbed by a user. However, liquid vapor generated by the conventional atomizer irritates a respiratory tract of a user, which affects user experience.

Documents US 2019/216135 A1 and WO 2020/209112 A1 disclose atomizers according to the preamble of the independent claim 1.

### SUMMARY

According to various exemplary embodiments of this application, an atomizer and an electronic atomization device including the atomizer are provided.

According to the invention, an atomizer is provided, including:
an ultrasonic atomization assembly including a liquid inlet surface and a first support surface surrounding the edge of the liquid inlet surface, where the first support surface and the liquid inlet surface are located on the same side of the ultrasonic atomization assembly; and
a heating assembly attached to the first support surface and configured to preheat the liquid approaching the liquid inlet surface.

According to the invention, the ultrasonic atomization assembly includes a piezoelectric ceramic piece and a metal piece, the liquid inlet surface and the first support surface are both located on a first side of the metal piece, a second side of the metal piece opposite the first side has a second support surface corresponding to the first support surface and a vapor outlet surface corresponding to the liquid inlet surface, the piezoelectric ceramic piece is attached to the second support surface and is provided with a through hole facing the vapor outlet surface, and the metal piece is provided with an atomization hole extending through the liquid inlet surface and the vapor outlet surface and in communication with the through hole.

In one of the embodiments, at least part of both the liquid inlet surface and the vapor outlet surface is a spherical crown surface, an opening of the spherical crown surface faces the first side, and the atomization hole is provided on the spherical crown surface.

In one of the embodiments, the heating assembly includes a heating layer, the heating layer is attached to the first side of the metal piece, and the heating layer is configured to include a high temperature zone surrounding the liquid inlet surface and a low temperature zone surrounding the high temperature zone.

In one of the embodiments, the distance between the edge of the liquid inlet surface and the heating layer is less than the distance between the edge of the first support surface and the heating layer. In one of the embodiments, the heating layer includes a first resistance wire surrounding the liquid inlet surface and a second resistance wire surrounding the first resistance wire, and the resistance of the first resistance wire is greater than the resistance of the second resistance wire.

In one of the embodiments, the heating assembly further includes a first insulating layer and a second insulating layer, the first insulating layer is attached to the first support surface, and the heating layer is sandwiched between the first insulating layer and the second insulating layer.

In one of the embodiments, the thermal conductivity of the second insulating layer is greater than the thermal conductivity of the first insulating layer.

In one of the embodiments, the heating assembly further includes a third insulating layer and a bonding layer, the heating layer is packaged in the third insulating layer, and the bonding layer is attached to the first support surface and is connected to the third insulating layer.

In one of the embodiments, the heating assembly is configured to be activated before the ultrasonic atomization assembly is activated.

In one of the embodiments, the atomization hole is a conical hole, and the bore diameter of the atomization hole is gradually reduced from the liquid inlet surface to the vapor outlet surface.

In one of the embodiments, the first insulating layer, the second insulating layer and the heating layer are provided with through holes in communication with each other, the through holes face the liquid inlet surface.

In one of the embodiments, the thickness of the first insulating layer and/or the second insulating layer ranges from 5 µm to 20 µm.

In one of the embodiments, the thickness of the heating layer ranges from 5 µm to 40 µm.

An electronic atomization device is provided, including the atomizer of any one of the above.

The heating assembly may preheat liquid to a temperature close to a body temperature, and when the ultrasonic atomization assembly atomizes the liquid to form liquid vapor, a temperature of the liquid vapor is close to the body temperature, so as to prevent the liquid vapor from irritating a respiratory tract of a human body and improve user experience. In addition, the heating assembly is directly attached to a back of the ultrasonic atomization assembly, that is, the heating assembly is integrated and arranged on the ultrasonic atomization assembly. Therefore, the heating assembly may heat liquid near the liquid inlet surface, i.e. a part of the liquid, so that heated liquid may directly enter the ultrasonic atomization assembly for atomization. In this way, the liquid is heated up quickly, energy consumption of the heating assembly is reduced, and time for waiting the liquid vapor to spray is reduced to improve the user experience. The heating assembly is directly arranged on the first support surface of the metal piece, that is, the heating assembly is located on a side on which the liquid inlet surface is located, so that the heating assembly can be in direct contact with the liquid, and heat generated by the heating assembly is directly transmitted to the liquid without an intermediate medium, thereby reducing heat loss, improving a heat utilization rate, making the liquid heat up quickly, and reducing time for waiting the liquid vapor to spray. In addition, there is no need to set an additional heat insulation part on the heating assembly, thus simplifying an overall structure of the atomizer.

### BRIEF DESCRIPTION OF THE DRAWINGS

To describe the technical solutions in the embodiments of this application or the conventional technology more clearly, the following outlines the drawings to be used in the description of the embodiments or the conventional technology. Evidently, the drawings outlined below are merely a part of embodiments of this application, and a person of ordinary skill in the art may derive other drawings from the outlined drawings without making any creative effort.
FIG. 1 is a schematic perspective view of an atomizer according to an embodiment;
FIG. 2 is a schematic perspective view of the atomizer shown in FIG. 1 from another angle of view;
FIG. 3 is a schematic exploded view of the atomizer shown in FIG. 1;
FIG. 4 is a schematic planar cross-sectional view of the atomizer shown in FIG. 1;
FIG. 5 is a partial schematic view of FIG. 4;
FIG. 6 is an enlarged schematic view of portion A in FIG. 5; and
FIG. 7 is a planar schematic cross-sectional view of an atomizer according to another embodiment.

### DETAILED DESCRIPTION

For ease of understanding this application, this application is described more comprehensively below with reference to the related accompanying drawings.

The accompanying drawings show exemplary implementations of this application. However, this application may be implemented in many different forms, and is not limited to the implementations described in this specification. On the contrary, the implementations are provided to make understanding of the disclosed content of this application more comprehensive.

It needs to be noted that, when a component is referred to as "being fixed to" another component, the component may be directly on the other component, or an intermediate component may be present. When a component is considered to be "connected to" another component, the component may be directly connected to the other component, or an intermediate component may also be present. The terms "inner", "outer", "left", "right" and similar expressions used in this specification are only for purposes of illustration but not indicate a unique implementation.

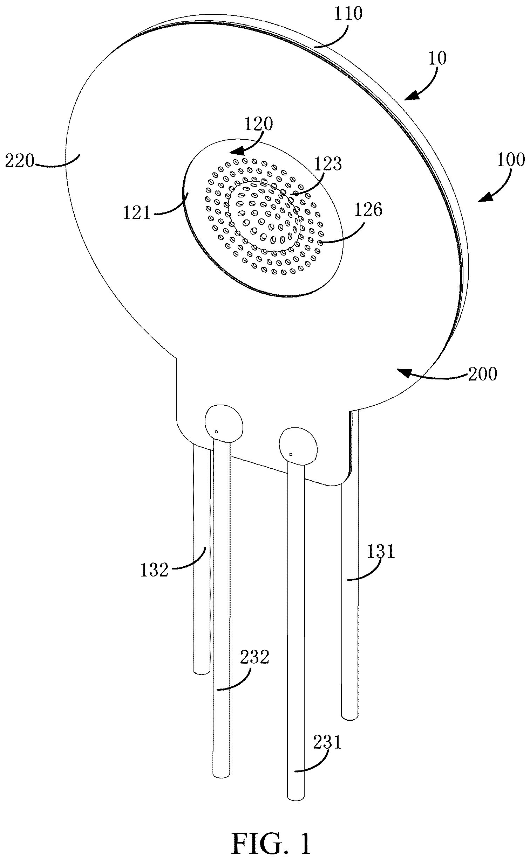

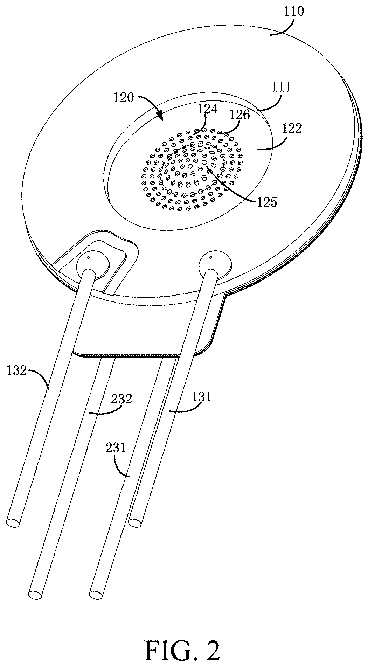

Referring to FIG. 1, FIG. 2, and FIG. 3, an atomizer 10 provided by an embodiment of this application includes an ultrasonic atomization assembly 100 and a heating assembly 200. The ultrasonic atomization assembly 100 is configured to atomize liquid to form liquid vapor, and the liquid may be oil, liquid medicine, or the like. When the liquid is atomized to form the liquid vapor, a user may inhale the liquid vapor.

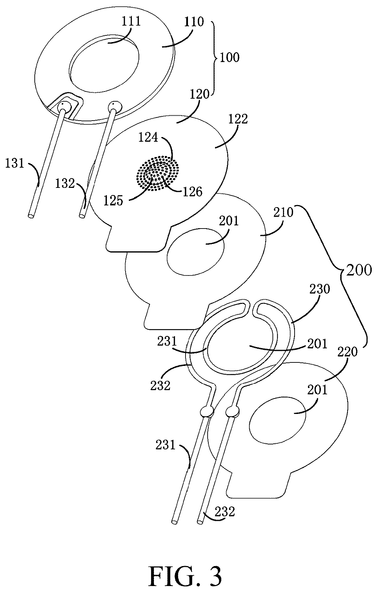

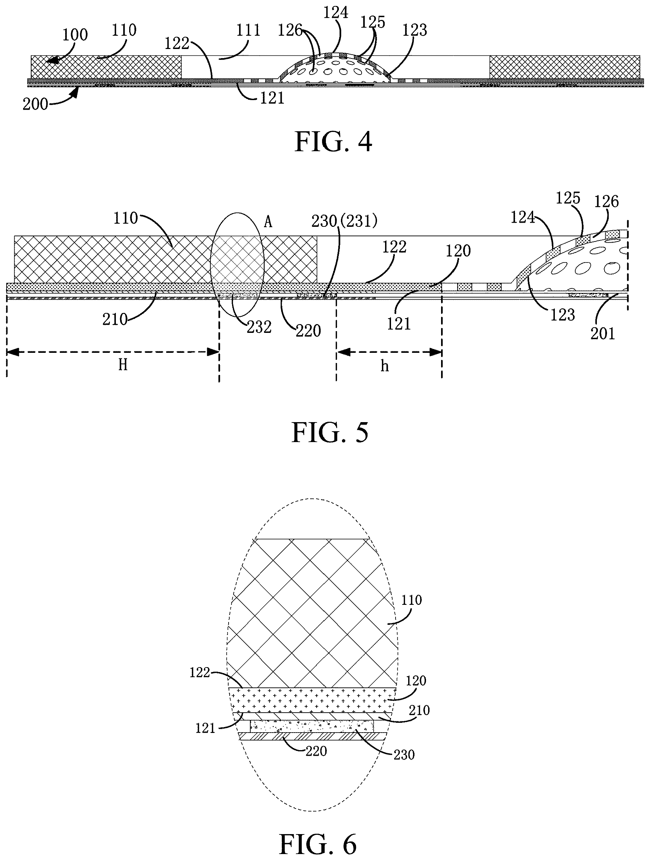

Referring to FIG. 3, FIG. 4, and FIG. 5, in some embodiments, the ultrasonic atomization assembly 100 includes a piezoelectric ceramic piece 110, a metal piece 120, a first electrode 131, and a second electrode 132. An end of the first electrode 131 and an end of the second electrode 132 are electrically connected to a circuit in the piezoelectric ceramic piece 110, and the other end of the first electrode 131 and the other end of the second electrode 132 are configured to be connected to an alternating current power supply. Therefore, the circuit in the piezoelectric ceramic piece 110 is configured to be supplied with an alternating current. When the alternating current power supply supplies the alternating current to the circuit in the piezoelectric ceramic piece 110 through the first electrode 131 and the second electrode 132, the piezoelectric ceramic piece 110 generates high frequency vibration, so that a vibration frequency of the piezoelectric ceramic piece 110 is equal to a vibration frequency of an ultrasound. A through hole 111 is provided at a center of the piezoelectric ceramic piece 110, and the through hole 111 penetrates two opposite surfaces of the piezoelectric ceramic piece 110. By providing the through hole 111, the piezoelectric ceramic piece 110 has a substantially circular structure.

The metal piece 120 has a substantially disk-shaped structure and includes a first support surface 121, a second support surface 122, a liquid inlet surface 123, and a vapor outlet surface 124. The liquid inlet surface 123 and the vapor outlet surface 124 are opposite to each other and are located at a central region of the metal piece 120. The second support surface 122 and the first support surface 121 are opposite to each other and are located at an edge region of the metal piece 120. The second support surface 122 and the vapor outlet surface 124 are located on a second side (i.e. an upper side) of the metal piece 120, and the second support surface 122 is connected to a periphery of the vapor outlet surface 124, so that the second support surface 122 is circular and surrounds the vapor outlet surface 124. The first support surface 121 and the liquid inlet surface 123 are located on a first side (i.e. a lower side) of the metal piece 120, and the first support surface 121 is connected to a periphery of the liquid inlet surface 123, so that the first support surface 121 is circular and surrounds the liquid inlet surface 123. In other words, both the second support surface 122 and the first support surface 121 are spaced apart along a thickness direction of the metal piece 120 and correspond to each other, and both the liquid inlet surface 123 and the vapor outlet surface 124 are spaced apart along the thickness direction of the metal piece 120 and correspond to each other.

A plurality of atomization holes 126 are provided on the metal piece 120, and the atomization holes 126 penetrate both the liquid inlet surface 123 and the vapor outlet surface 124. The heating assembly 200 is arranged on the first support surface 121 of the metal piece 120. The piezoelectric ceramic piece 110 is attached to the second support surface 122 of the metal piece 120, so that the vapor outlet surface 124 corresponds to the through hole 111 of the piezoelectric ceramic piece 110, and the through hole 111 is in communication with the atomization hole 126. Liquid to be atomized is located on a side at which the liquid inlet surface 123 is located, and the liquid is in direct contact with the liquid inlet surface 123, so that the liquid enters the atomization hole 126 through the liquid inlet surface 123. When the piezoelectric ceramic piece 110 generates high frequency vibration under an action of an alternating current, vibration energy of the piezoelectric ceramic piece 110 is transmitted to the metal piece 120, so that the metal piece 120 also generates high frequency vibration along with the piezoelectric ceramic piece 110, so as to atomize the liquid in the atomization hole 126 to form liquid vapor. The liquid vapor is sprayed out from the vapor outlet surface 124 into the through hole 111 of the piezoelectric ceramic piece 110 to be inhaled by a user. In some embodiments, the atomization hole 126 is a conical hole, and a diameter of the atomization hole 126 is gradually reduced from the liquid inlet surface 123 to the vapor outlet surface 124.

The metal piece 120 may be made of a stainless steel material, so that the metal piece 120 has good structural strength, thermal conductivity, and rust resistance. Therefore, it can be ensured that the metal piece 120 has sufficient anti-fatigue strength, which prevents fatigue fracture of the metal piece 120 under high frequency vibration, and improves a service life of the entire ultrasonic atomization assembly 100. In addition, it can prevent a rust particle from partially or completely blocking the atomization hole 126, ensure that particle sizes of tiny liquid droplets in the liquid vapor are equal, and ensure that each atomization hole 126 can atomize the liquid, so as to finally improve uniformity and reliability of the metal piece 120 in vaporizing the liquid.

In some embodiments, at least a part of both the liquid inlet surface 123 and the vapor outlet surface 124 is a spherical crown surface 125. An opening of the spherical crown surface 125 faces the heating assembly 200, so that an entire central region of the metal piece 120 forms a spherical crown protrusion, and an opening of the protrusion is the opening of the spherical crown surface 125. The protrusion may also be formed by a central region of a flat metal piece 120 recessed toward the first side. Another part of the atomization hole 126 may be located on the spherical crown surface 125, and another part of the atomization hole 126 may be located at other parts of the liquid inlet surface 123 and the vapor outlet surface 124. Certainly, all atomization holes 126 may be located on the spherical crown surface 125. By providing the spherical crown surface 125, a plane perpendicular to a thickness direction of the metal piece 120 is used as a reference plane. Although an orthographic projection of a metal piece 120 having the spherical crown surface 125 on the reference plane is the same as that of the flat metal piece 120 on the reference plane, the metal piece 120 having the spherical crown surface 125 can ensure that the liquid vapor is sprayed in different directions and has a relatively large spraying range. In addition, a relatively large number of atomization holes 126 may be provided, so that an atomization amount of liquid per unit time is increased to increase liquid vapor concentration.

Referring to FIG. 3, FIG. 5, and FIG. 6, in some embodiments, the heating assembly 200 includes a first insulating layer 210, a second insulating layer 220, and a heating layer 230. The first insulating layer 210, the second insulating layer 220, and the heating layer 230 are all substantially circular, and are provided with through holes 201 in communication with each other. The through holes 201 correspond to the liquid inlet surface 123 of the metal piece 120, and liquid may be in contact with the liquid inlet surface 123 through the through holes 201 and enter the atomization hole 126 to be atomized. The first insulating layer 210 is attached to the first support surface 121 of the metal piece 120, and the first insulating layer 210 may be attached to the first support surface 121 through a physical vapor deposition (PVD) process or a screen printing process, so that the first insulating layer 210 is directly connected to the metal piece 120, so as to prevent the first insulating layer 210 from being connected to the metal piece 120 through another connection layer, and reduce a thickness and weight of the entire atomizer 10, which facilitates a thin and light design of the atomizer 10. The thickness of the first insulating layer 210 may range from 5 µm to 20 µm, for example, a specific value may be 5 µm, 10 µm, 15 µm, 20 µm, or the like. On the premise of ensuring that the first insulating layer 210 has sufficient insulating performance, the thickness of the first insulating layer 210 may be appropriately reduced, so that the thickness of the entire atomizer 10 may be further compressed.

The heating layer 230 is attached to a surface of the first insulating layer 210 away from the metal piece 120, and the heating layer 230 may also be attached to the first insulating layer 210 through the physical vapor deposition (PVD) process or the screen printing process, so that the heating layer 230 is directly connected to the first insulating layer 210, to prevent the heating layer 230 from being connected to the first insulating layer 210 through another connecting layer, which may also reduce the thickness of the entire atomizer 10 and realize the thin and light design of the atomizer 10. The thickness of the heating layer 230 may range from 5 µm to 40 µm, for example, a specific value may be 5 µm, 20 µm, 30 µm, 40 µm, or the like. The heating layer 230 may further include a third electrode 231 and a fourth electrode 232. One end of the third electrode 231 and one end of the fourth electrode 232 are electrically connected to the heating layer 230, and the other end of the third electrode 231 and the other end of the fourth electrode 232 are configured to be connected to a direct current power supply. Therefore, the heating layer 230 is configured to be supplied with direct current. The heating layer 230 may convert electrical energy to heat energy when the direct current power supply (e.g. a battery) applies a direct current to the heating layer 230 through the third electrode 231 and the fourth electrode 232. The heating layer 230 may be a layered structure formed by bending a wire-like wire or directly be a layered structure formed by a conductive diaphragm. The heating layer 230 may be a carbon nanoflake or may be made of a metal or alloy material such as a stainless steel material, a titanium metal material, or a titanium alloy material.

The second insulating layer 220 is attached to a surface of the heating layer 230 away from the metal piece 120, and the second insulating layer 220 may also be attached to the heating layer 230 through the physical vapor deposition (PVD) process or the screen printing process, so that the heating layer 230 is directly connected to the second insulating layer 220, to prevent the heating layer 230 from being connected to the second insulating layer 220 through another connecting layer, which may also reduce the thickness of the entire atomizer 10 and realize the thin and light design of the atomizer 10. The thickness of the second insulating layer 220 may range from 5 µm to 20 µm, for example, a specific value may be 5 µm, 10 µm, 15 µm, 20 µm, or the like. On the basis of ensuring that the second insulating layer 220 has sufficient insulating performance, the thickness of the second insulating layer 220 may be appropriately reduced, so that the thickness of the entire atomizer 10 may be further compressed. The second insulating layer 220 may be made of a ceramic glaze material, so that the second insulating layer 220 has high wear resistance and good thermal conductivity.

Therefore, for the entire heating assembly 200, the first insulating layer 210 is arranged on the first support surface 121 of the metal piece 120, and the heating layer 230 is directly sandwiched between the first insulating layer 210 and the second insulating layer 220. By providing the first insulating layer 210, a short circuit phenomenon caused by a direct contact between the heating layer 230 and the metal piece 120 may be avoided. By providing the second insulating layer 220, the second insulating layer 220 is in direct contact with liquid on a side of the liquid inlet surface 123, so as to avoid a short circuit phenomenon caused by a direct contact between the heating layer 230 and the liquid, and also avoid contamination on the liquid caused by a contact between the heating layer 230 and the liquid.

During operation of the atomizer 10, the heating assembly 200 is activated before the ultrasonic atomization assembly 100 is activated. Specifically, a direct current power supply is used for supplying power to the heating layer 230 through the third electrode 231 and the fourth electrode 232, and then an alternating current power supply is used for supplying power to the circuit in the piezoelectric ceramic piece 110 through the first electrode 131 and the second electrode 132, so that a working time of the heating layer 230 is earlier than a working time of the piezoelectric ceramic piece 110. For example, the heating layer 230 may be activated no more than one second before the piezoelectric ceramic piece 110 is activated, so that the piezoelectric ceramic piece 110 can drive the metal piece 120 to vibrate to atomize liquid to form liquid vapor as soon as possible, which reduces time of waiting for the liquid vapor by a user, and improves user experience of the entire atomizer 10.

By activating the heating assembly 200 first, the heating assembly 200 can preheat the liquid approaching the liquid inlet surface 123. Certainly, the heating assembly 200 may preheat the liquid to a temperature close to a body temperature. When the metal piece 120 atomizes the liquid to form liquid vapor sprayed from the vapor outlet surface 124 into the through hole 111, the temperature of the liquid vapor is close to the body temperature. Especially for atomization of liquid medicine, when the user inhales the liquid medicine that has a temperature close to the body temperature and exists in a form of liquid vapor, the liquid medicine (liquid vapor) can be prevented from irritating a respiratory tract of a human body, so as to avoid other symptoms such as cough or elevated blood pressure, and ensure a therapeutic effect of the liquid medicine on the user. In addition, a temperature difference between the liquid vapor in an early stage and a middlelate stage of the heating assembly 200 due to simultaneous activating of the heating assembly 200 and the piezoelectric ceramic piece 110 may be eliminated, the temperature of the liquid vapor in an atomization process may be kept consistent, and comfort of inhaling the liquid vapor may be improved. Further, for liquid with relatively high viscosity, through a preheating action of the heating assembly 200, the viscosity of the liquid can be appropriately reduced, thereby increasing fluidity of the liquid, making it easier for the liquid to enter the atomization hole 126 and to be rapidly atomized to form liquid vapor under high frequency vibration, thus avoiding a phenomenon that the liquid is difficult to flow into the atomization hole 126 or is difficult to be atomized to form the liquid vapor, and improving atomization efficiency, atomization stability, and atomization reliability of the entire atomizer 10.

If a separate heating assembly 200 is used for heating the liquid, the heating assembly 200 simultaneously heat liquid near the liquid inlet surface 123 and liquid far away from the liquid inlet surface 123. When the liquid far away from the liquid inlet surface 123 reaches near the liquid inlet surface 123, the heating assembly 200 repeatedly heats the liquid, resulting in a waste of energy. In addition, due to a large amount of heated liquid, the liquid is heated up slowly, which also prolongs waiting time of the user. In the foregoing embodiment, the heating assembly 200 is directly attached to the first support surface 121 of the ultrasonic atomization assembly 100, that is, the heating assembly 200 is integrated and arranged on the ultrasonic atomization assembly 100. Therefore, the heating assembly 200 may merely heat liquid near the liquid inlet surface 123, i.e. a part of the liquid, so that heated liquid may directly enter the atomization hole 126 for atomization. In this way, the liquid is heated up quickly, energy consumption of the heating assembly 200 is reduced, and time for waiting the liquid vapor to spray is reduced to improve the user experience.

If the heating assembly 200 is arranged on a side on which the vapor outlet surface 124 of the metal piece 120 is located, the heating assembly 200 cannot be in direct contact with liquid on a side of the liquid inlet surface 123, and heat generated by the heating assembly 200 is transferred to the liquid through the metal piece 120 to preheat the liquid, thus prolonging a heat transfer path, increasing a heat loss generated in a heat transfer process, and reducing a heat utilization rate of the heating assembly 200. In addition, the heating assembly 200 needs to be protected by a heat insulation part, to prevent heat of the heating assembly 200 from being transferred to another member of the atomizer 10 and causing damage to the member. Therefore, arrangement of the heat insulation part significantly complicates a structure of the atomizer 10. However, in the foregoing embodiment, the entire heating assembly 200 is directly arranged on the first support surface 121 of the metal piece 120, that is, the heating assembly 200 is located on a side on which the liquid inlet surface 123 is located, so that the heating assembly 200 can be in direct contact with the liquid, and heat generated by the heating assembly 200 is directly transmitted to the liquid without an intermediate medium, thereby reducing heat loss, improving a heat utilization rate, making the liquid heat up quickly, and reducing time for waiting the liquid vapor to spray. In addition, there is no need to set an additional heat insulation part on the heating assembly 200, thus simplifying an overall structure of the atomizer 10.

In some embodiments, the heating layer 230 is configured to include a high temperature zone surrounding the liquid inlet surface 123 and a low temperature zone surrounding the high temperature zone, so that the high temperature zone of the heating layer 230 may be closer to the liquid inlet surface 123 than the low temperature zone. In some embodiments, the heating layer 123 includes a first resistance wire 231 surrounding the liquid inlet surface 123 and a second resistance wire 232 surrounding the first resistance wire. A resistance of the first resistance wire 231 is greater than a resistance of the second resistance wire 232. Because the first resistance wire 231 and the second resistance wire 232 are connected in series, heat generated by the first resistance wire 231 is more than heat generated by the second resistance wire 232 in the same time period, so that the first resistance wire 231 corresponds to the high temperature zone and the second resistance wire 232 corresponds to the low temperature zone. Therefore, heat generated by the heating layer 230 is more focused on the liquid near the liquid inlet surface 123, which may reduce repeated heating and increase a heating speed of the liquid. In addition, heat transferred by the heating layer 230 to another member of the atomizer 10 through an edge of the metal piece 120 may be reduced and an energy utilization rate of the heating layer 230 may be improved. In other embodiments, in a case that the resistance of the first resistance wire 231 is equal to the resistance of the second resistance wire 232, the liquid inlet surface 123 may be arranged closer to the heating layer 230 relative to an edge of the first support surface 121 of the metal piece 120. In other words, in a case that a distance h between the heating layer 230 and the liquid inlet surface 123 is less than a distance H between the heating layer 230 and the edge of the first support surface 121, the heat generated by the heating layer 230 may also be more focused on the liquid near the liquid inlet surface 123, thus improving the energy utilization rate of the heating layer 230. A thermal conductivity of the second insulating layer 220 is greater than a thermal conductivity of the first insulating layer 210, so that the heat generated by the heating layer 230 may be easily and directly transferred to the liquid through the second insulating layer 220, heat transferred to another member of the atomizer 10 through the first insulating layer 210 and the metal piece 120 may be reduced, and the energy utilization rate of the heating layer 230 may also be improved.

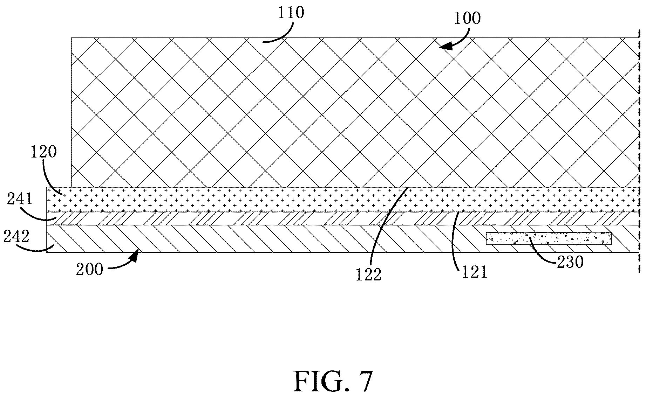

Referring to FIG. 7, in some embodiments, during a manufacturing process of the heating assembly 200, for example, a prepared heating layer 230 may be placed in an injection mold and then a liquid insulating material is injected to a cavity of the mold. The liquid insulating material may be cooled and solidified to form an insulating layer, and the insulating layer may be denoted as a third insulating layer 241. Therefore, the entire heating layer 230 is packaged in the third insulating layer 241. By the action of the third insulating layer 241, short circuit caused by a contact between the heating layer 230 and the metal piece 120 as well as the liquid can be avoided. In some embodiments, the third insulating layer 241 may be provided with a slot structure, so that a third electrode 231 and a fourth electrode 232 electrically connected to the heating layer 230 penetrate the slot structure. During a mounting process of the heating assembly 200, a bonding layer 242 may be provided on the first support surface 121 of the metal piece 120, and the third insulating layer 241 is attached to the bonding layer 242. That is, the third insulating layer 241 is arranged on the first support surface 121 of the metal piece 120 through the bonding layer 242. Certainly, the third insulating layer 241 may be fixed on the first support surface 121 of the metal piece 120 through a snap connection. In another example, an insulating layer may be provided on both sides of the prepared heating layer 230 through physical vapor deposition, electrophoresis, or spraying, and then the insulating layer is fixed on the metal piece 120 through the bonding layer 242 or the snap connection.

This application further provides an electronic atomization device. The electronic atomization device includes the forgoing atomizer 10. By arranging the atomizer 10, the user experience of the electronic atomization device can be improved, energy consumption can be reduced, and the structure can be thinner and lighter.

## Claims

1. An atomizer (10), comprising:
an ultrasonic atomization assembly (100) comprising a liquid inlet surface (123) and a first support surface (121) surrounding an edge of the liquid inlet surface (123), wherein the first support surface (121) and the liquid inlet surface (123) are located on the same side of the ultrasonic atomization assembly (100); and
a heating assembly (200) attached to the first support surface (121) and configured to preheat the liquid approaching the liquid inlet surface (123),
**characterized in that** the ultrasonic atomization assembly (100) comprises a piezoelectric ceramic piece (110) and a metal piece (120), the liquid inlet surface (123) and the first support surface (121) are both located on a first side of the metal piece (120), a second side of the metal piece (120) opposite the first side has a second support surface (122) corresponding to the first support surface (121) and a vapor outlet surface (124) corresponding to the liquid inlet surface (123), the piezoelectric ceramic piece (110) is attached to the second support surface (122) and is provided with a through hole (111) facing the vapor outlet surface (124), and the metal piece (120) is provided with an atomization hole (126) extending through the liquid inlet surface (123) and the vapor outlet surface (124) and in communication with the through hole (111).

2. The atomizer (10) of claim 1, wherein at least part of both the liquid inlet surface (123) and the vapor outlet surface (124) is a spherical crown surface (125), an opening of the spherical crown surface (125) faces the first side, and the atomization hole (126) is provided on the spherical crown surface (125).

3. The atomizer (10) of claim 1, wherein the heating assembly (200) comprises a heating layer (230) attached to the first side of the metal piece (120), and the heating layer (230) is configured to comprise a high temperature zone surrounding the liquid inlet surface (123) and a low temperature zone surrounding the high temperature zone, and wherein the heating assembly (200) further comprises a first insulating layer (210) and a second insulating layer (220), the first insulating layer (210) is attached to the first support surface (121), and the heating layer (230) is sandwiched between the first insulating layer (210) and the second insulating layer (220).

4. The atomizer (10) of claim 3, wherein a distance between the edge of the liquid inlet surface (123) and the heating layer (230) is less than the distance between an edge of the first support surface (121) and the heating layer (230).

5. The atomizer (10) of claim 3, wherein the heating layer (230) comprises a first resistance wire (231) surrounding the liquid inlet surface (123) and a second resistance wire (232) surrounding the first resistance wire (231), and a resistance of the first resistance wire (231) is greater than a resistance of the second resistance wire (232).

6. The atomizer (10) of claim 3, wherein a thermal conductivity of the second insulating layer (220) is greater than a thermal conductivity of the first insulating layer (210).

7. The atomizer (10) of claim 3, wherein the heating assembly (200) further comprises a third insulating layer (241) and a bonding layer (242), the heating layer (230) is packaged in the third insulating layer (241), and the bonding layer (242) is attached to the first support surface (121) and is connected to the third insulating layer (241).

8. The atomizer (10) of any one of the preceding claims, wherein the heating assembly (200) is configured to be activated before the ultrasonic atomization assembly (100) is activated.

9. The atomizer (10) of claim 1, wherein the atomization hole (126) is a conical hole, and a bore diameter of the atomization hole (126) is gradually reduced from the liquid inlet surface (123) to the vapor outlet surface (124).

10. The atomizer (10) of claim 3, wherein the first insulating layer (210), the second insulating layer (220), and the heating layer (230) are provided with through holes (201) in communication with each other, the through holes face the liquid inlet surface.

11. The atomizer (10) of claim 3, wherein a thickness of the first insulating layer (210) and/or the second insulating layer (220) ranges from 5 µm to 20 µm.

12. The atomizer (10) of claim 3, wherein the thickness of the heating layer (230) ranges from 5 µm to 40 µm.

13. An electronic atomization device, comprising the atomizer (10) of any one of claims 1 to 12.

## Patentansprüche

1. Zerstäuber (10), umfassend:
eine Ultraschallzerstäubungsanordnung (100), umfassend eine Flüssigkeitseinlassfläche (123) und eine erste Trägerfläche (121) umfasst, die einen Rand der Flüssigkeitseinlassfläche (123) umgibt, wobei sich die erste Trägerfläche (121) und die Flüssigkeitseinlassfläche (123) auf derselben Seite der Ultraschallzerstäubungsanordnung (100) befinden; und
eine Heizanordnung (200), die an der ersten Trägerfläche (121) angebracht und konfiguriert ist, um die Flüssigkeit vorzuwärmen, die sich der Flüssigkeitseinlassfläche (123) nähert,
**dadurch gekennzeichnet, dass** die Ultraschallzerstäubungsanordnung (100) ein piezoelektrisches Keramikteil (110) und ein Metallteil (120) umfasst, sich die Flüssigkeitseinlassfläche (123) und die erste Trägerfläche (121) beide auf einer ersten Seite des Metallteils (120) befinden, eine zweite Seite des Metallteils (120) gegenüber der ersten Seite eine zweite Trägerfläche (122), die der ersten Trägerfläche (121) entspricht, und eine Dampfauslassfläche (124), die der Flüssigkeitseinlassfläche (123) entspricht, aufweist, das piezoelektrische Keramikteil (110) an der zweiten Trägerfläche (122) angebracht und mit einem Durchgangsloch (111) versehen ist, das der Dampfauslassfläche (124) zugewandt ist, und das Metallteil (120) mit einem Zerstäubungsloch (126) versehen ist, das sich durch die Flüssigkeitseinlassfläche (123) und die Dampfauslassfläche (124) erstreckt und mit dem Durchgangsloch (111) in Verbindung ist.

2. Zerstäuber (10) nach Anspruch 1, wobei zumindest ein Teil von sowohl der Flüssigkeitseinlassfläche (123) als auch der Dampfauslassfläche (124) eine kugelförmige Kronenfläche (125) ist, eine Öffnung der kugelförmigen Kronenfläche (125) der ersten Seite zugewandt ist, und das Zerstäubungsloch (126) an der kugelförmigen Kronenfläche (125) bereitgestellt ist.

3. Zerstäuber (10) nach Anspruch 1, wobei die Heizanordnung (200) eine Heizschicht (230) umfasst, die an der ersten Seite des Metallteils (120) angebracht ist, und die Heizschicht (230) konfiguriert ist, um eine Hochtemperaturzone, die die Flüssigkeitseintrittsfläche (123) umgibt, und eine Niedertemperaturzone, die die Hochtemperaturzone umgibt, zu umfassen, und wobei die Heizanordnung (200) ferner eine erste Isolierschicht (210) und eine zweite Isolierschicht (220) umfasst, die erste Isolierschicht (210) an der ersten Trägerfläche (121) angebracht ist, und die Heizschicht (230) zwischen die erste Isolierschicht (210) und die zweite Isolierschicht (220) eingefügt ist.

4. Zerstäuber (10) nach Anspruch 3, wobei ein Abstand zwischen dem Rand der Flüssigkeitseinlassfläche (123) und der Heizschicht (230) geringer ist als der Abstand zwischen einem Rand der ersten Trägerfläche (121) und der Heizschicht (230).

5. Zerstäuber (10) nach Anspruch 3, wobei die Heizschicht (230) einen ersten Widerstandsdraht (231), der die Flüssigkeitseinlassfläche (123) umgibt, und einen zweiten Widerstandsdraht (232), der den ersten Widerstandsdraht (231) umgibt, umfasst, und ein Widerstand des ersten Widerstandsdrahts (231) größer ist als ein Widerstand des zweiten Widerstandsdrahts (232).

6. Zerstäuber (10) nach Anspruch 3, wobei eine Wärmeleitfähigkeit der zweiten Isolierschicht (220) größer ist als eine Wärmeleitfähigkeit der ersten Isolierschicht (210).

7. Zerstäuber (10) nach Anspruch 3, wobei die Heizanordnung (200) ferner eine dritte Isolierschicht (241) und eine Haftschicht (242) umfasst, die Heizschicht (230) in der dritten Isolierschicht (241) eingekapselt ist, und die Haftschicht (242) an der ersten Trägerfläche (121) angebracht und mit der dritten Isolierschicht (241) verbunden ist.

8. Zerstäuber (10) nach einem der vorherigen Ansprüche, wobei die Heizanordnung (200) konfiguriert ist, um aktiviert zu werden, bevor die Ultraschallzerstäubungsanordnung (100) aktiviert wird.

9. Zerstäuber (10) nach Anspruch 1, wobei das Zerstäubungsloch (126) ein konisches Loch ist und ein Bohrungsdurchmesser des Zerstäubungslochs (126) von der Flüssigkeitseinlassfläche (123) zu der Dampfauslassfläche (124) allmählich reduziert ist.

10. Zerstäuber (10) nach Anspruch 3, wobei die erste Isolierschicht (210), die zweite Isolierschicht (220) und die Heizschicht (230) mit Durchgangslöchern (201) in Verbindung miteinander versehen sind, wobei die Durchgangslöcher der Flüssigkeitseinlassfläche zugewandt sind.

11. Zerstäuber (10) nach Anspruch 3, wobei eine Stärke der ersten Isolierschicht (210) und/oder der zweiten Isolierschicht (220) in einem Bereich von 5 µm bis 20 µm ist.

12. Zerstäuber (10) nach Anspruch 3, wobei die Stärke der Heizschicht (230) in einem Bereich von 5 µm bis 40 µm ist.

13. Elektronische Zerstäubungsvorrichtung, umfassend den Zerstäuber (10) nach einem der Ansprüche 1 bis 12.

## Revendications

1. Atomiseur (10) comprenant :
un ensemble d'atomisation par ultrasons (100) comprenant une surface d'entrée de liquide (123) et une première surface d'appui (121) entourant un bord de la surface d'entrée de liquide (123), où se trouvent la première surface d'appui (121) et la surface d'entrée de liquide (123) du même côté de l'ensemble d'atomisation par ultrasons (100); et
un ensemble chauffant (200) fixé à la première surface d'appui (121) et configuré pour préchauffer le liquide qui approche de la surface d'entrée de liquide (123),
**caractérisé en ce que** l'ensemble d'atomisation par ultrasons (100) comprend une pièce de céramique piézoélectrique (110) et une pièce métallique (120), la surface d'entrée de liquide (123) et la première surface d'appui (121) sont toutes deux situées sur un premier côté de la pièce métallique (120), un deuxième côté de la pièce métallique (120) en face du premier côté a une deuxième surface d'appui (122) correspondant à la première surface d'appui (121) et une surface de sortie de la vapeur (124) correspondant à la surface d'entrée de liquide (123), la pièce de céramique piézoélectrique (110) est fixée à la deuxième surface d'appui (122) et comprend un trou débouchant (111) faisant face à la surface de sortie de la vapeur (124), et la pièce métallique (120) est fournie avec un trou d'atomisation (126) s'étendant par la surface d'entrée de liquide (123) et la surface de sortie de la vapeur (124) et en communication avec le trou débouchant (111).

2. Atomiseur (10) selon la revendication 1, dans lequel au moins une partie de la surface d'entrée de liquide (123) et de la surface de sortie de la vapeur (124) est une surface à couronne sphérique (125), une ouverture de la surface à couronne sphérique (125) fait face au premier côté, et le trou d'atomisation (126) est prévu sur la surface à couronne sphérique (125).

3. Atomiseur (10) selon la revendication 1, dans lequel l'ensemble chauffant (200) comprend une couche chauffante (230) fixée au premier côté de la pièce métallique (120), et la couche chauffante (230) est configurée pour comprendre une zone à haute température entourant la surface d'entrée de liquide (123) et une zone à basse température entourant la zone à haute température, où l'ensemble chauffant (200) comprend en outre une première couche isolante (210) et une deuxième couche isolante (220), la première couche isolante (210) est fixée à la première surface d'appui (121) et la couche chauffante (230) est prise en sandwich entre la première couche isolante (210) et la deuxième couche isolante (220).

4. Atomiseur (10) selon la revendication 3, dans lequel une distance entre le bord de la surface d'entrée de liquide (123) et la couche chauffante (230) est inférieure à la distance entre un bord de la première surface d'appui (121) et la couche chauffante (230).

5. Atomiseur (10) selon la revendication 3, dans lequel la couche chauffante (230) comprend un premier fil de résistance (231) entourant la surface d'entrée de liquide (123) et un deuxième fil de résistance (232) entourant le premier fil de résistance (231), et une résistance du premier fil de résistance (231) est supérieure à une résistance du deuxième fil de résistance (232).

6. Atomiseur (10) selon la revendication 3, dans lequel la conductivité thermique de la deuxième couche isolante (220) est supérieure à celle de la première couche isolante (210).

7. Atomiseur (10) selon la revendication 3, dans lequel l'ensemble chauffant (200) comprend en outre une troisième couche isolante (241) et une couche de liaison (242), la couche chauffante (230) est emballée dans la troisième couche isolante (241), et la couche de liaison (242) est fixée à la première surface d'appui (121) et est relié à la troisième couche isolante (241).

8. Atomiseur (10) selon l'une quelconque des revendications précédentes, dans lequel l'ensemble chauffant (200) est configuré pour être activé avant l'ensemble d'atomisation par ultrasons (100).

9. Atomiseur (10) selon la revendication 1, dans lequel le trou d'atomisation (126) est un trou conique, et le diamètre du trou d'atomisation (126) est graduellement réduit de la surface d'entrée de liquide (123) à la surface de sortie de la vapeur (124).

10. Atomiseur (10) selon la revendication 3, dans lequel la première couche isolante (210), la deuxième couche isolante (220) et la couche chauffante (230) sont fournies avec des trous débouchants (201) qui sont en communication les uns avec les autres, les trous débouchants font face à la surface d'entrée de liquide.

11. Atomiseur (10) selon la revendication 3, dans lequel l'épaisseur de la première couche isolante (210) et/ou de la deuxième couche isolante (220) varie de 5 µm à 20 µm.

12. Atomiseur (10) selon la revendication 3, dans lequel l'épaisseur de la couche chauffante (230) varie de 5 µm à 40 µm.

13. Dispositif électronique d'atomisation, comprenant l'atomiseur (10) selon l'une quelconque des revendications 1 à 12.
